# EUROPEAN PATENT APPLICATION

(11) **EP 3 015 091 A1**
(43) Date of publication of application: **04.05.2016**
(21) Application number: 14817374.3
(22) Date of filing: 24.06.2014
(51) Int. Cl.: A61F 2/10, C07K 14/78

(54) **PATCH-TYPE ARTIFICIAL SKIN PREPARATION**

(30) Priority: 28.06.2013 JP 2013136118
(71) Applicant: Saga University, Saga-shi, Saga 840-8502 (JP); Yutoku Pharmaceutical Industries Co., Ltd., Kashima-shi, Saga 849-1393 (JP)
(72) Inventor: AOKI, Shigehisa, Saga-shi Saga 840-8502 (JP); TAKEZAWA, Toshiaki, Tsukuba-shi Ibaraki 305-8602 (JP); MIYAZAKI, Ayumi, Tsukuba-shi Ibaraki 305-8602 (JP); HIRAYAMA, Hiroshi, Kashima-shi Saga 849-1393 (JP)
(74) Representative: Blodig, Wolfgang
(86) International application number: PCT/JP2014/066635
(87) International publication number: WO 2014/208525

(57) **Abstract**

The present invention addresses the problem of providing an artificial skin preparation which requires no suturing or the like when treating a wound area with a dried collagen vitrigel membrane material as an artificial skin, and also is less likely to cause contamination or the like with an exudate, and is free from the risk of secondary damage by replacement thereof. The artificial skin preparation for solving the problem includes at least an adhesive film, an adhesion-preventing sheet, and a dried collagen vitrigel membrane material in this order, and is characterized in that the adhesion-preventing sheet has a sufficient size for preventing the adhesion of the collagen vitrigel membrane to an adhesive layer of the adhesive film and is attached to the adhesive layer of the adhesive film at a position corresponding to the position of the dried collagen vitrigel membrane material, and the dried collagen vitrigel membrane material is made easily detachable from the adhesion-preventing sheet when the adhesive film is peeled off while fixing and holding the dried collagen vitrigel membrane material on the surface of the adhesion-preventing sheet during attachment.

## Description

### Technical Field

The present invention relates to a patch-type artificial skin preparation, and more particularly relates to a patch-type artificial skin preparation, which uses a dried collagen vitrigel membrane material, and can be easily attached to an affected area, and also does not cause damage to the collagen vitrigel membrane even when an adhesive material covering the dried collagen vitrigel membrane material is peeled off after the dried collagen vitrigel membrane material is fused to the affected area to some extent.

### Background Art

When all layers of the skin are lost by a wound due to a burn or the like, the regeneration of epidermal cells is most important. However, for the regeneration of epidermal cells, it is necessary to regenerate fibroblasts, which are feeder cells therefor, in the dermis. At the same time, fibroblasts play a role in producing an extracellular matrix, that is, collagen in the dermis, and regulate the microenvironment. In general, at a wound site, fibroblasts are transformed to myofibroblasts, and produce collagen. However, the myofibroblasts have a very high contractile activity and develop pathological contraction at a wound site to cause the formation of a keloid or a hypertrophic scar. Further, also in the case where skin tissue is lost to the deep dermis due to a burn, a scar may sometimes be formed in healed tissue by the same mechanism as traumatic injury, and in particular, serious cases in terms of aesthetic outcome for women and children are often observed. The improvement of the aesthetic outcome after wound healing has been demanded, however, a solution has not been established yet.

At present, an artificial skin is used for suppressing scar formation while promoting wound healing, and for example, PELNAC (Smith & Nephew) in which sponge-like collagen and a silicone membrane are combined, TERUDERMIS (Olympus Terumo Biomaterials Corporation), and the like are provided.

However, a currently available artificial skin is in a sponge form and is thick, so that the adhesiveness to a wound area is poor, and therefore, a complicated surgical treatment such as suturing is required when it is fixed to a wound area. Further, due to the thickness of the product, it has a problem that it is difficult to perform a drainage treatment of an exudate.

### Citation List

### Patent Literature

PTL 1: WO 2012/026531 A1

### Non Patent Literature

NPL 1: "Seibutsu-kogaku Kaishi", 2013, No. 4, pp. 214-217

### Summary of Invention

### Technical Problem

The present invention has been made in view of the above circumstances and addresses the problem of providing an artificial skin preparation which requires no suturing or the like when treating a wound area with a dried collagen vitrigel membrane material as an artificial skin, and also is less likely to cause contamination or the like with an exudate, and is free from the risk of secondary damage by replacement thereof.

### Solution to Problem

The present inventors studied the improvement of conventional artificial skins, and as a result, they found first that wound healing is promoted and also scar formation can be suppressed by suturing and fixing a material including a dried material (collagen xerogel membrane) obtained by drying a collagen vitrigel membrane and a polyvinylidene chloride wrap in combination to a wound area where the skin is lost.

Then, in order to perform this method more simply, an adhesive layer is provided on a wrap film, and a dried collagen vitrigel membrane material is placed thereon to cover a wound area where the skin is lost. In this method, the handling became easy, however, it became clear that when the wrap film is peeled off after attachment, the collagen vitrigel is also peeled off from the tissue, so that regenerated tissue which has migrated, infiltrated, and proliferated inside and outside the vitrigel may be damaged to impair the regeneration at the wound site.

Therefore, a method in which a dried collagen vitrigel membrane material is stably fixed to an area where the skin is lost, and also even when the adhesive film is peeled off, the attached collagen vitrigel membrane is not damaged was intensively studied. As a result, it was found that the above problem can be solved by inserting an adhesion-preventing film between a dried collagen vitrigel membrane material and an adhesive layer of an adhesive film, and thus, the present invention was completed.

That is, the present invention is directed to a patch-type artificial skin preparation including at least an adhesive film, an adhesion-preventing sheet, and a dried collagen vitrigel membrane material in this order, characterized in that the adhesion-preventing sheet has a sufficient size for preventing the adhesion of the dried collagen vitrigel membrane material to an adhesive layer of the adhesive film and is attached to the adhesive layer of the adhesive film at a position corresponding to the position of the dried collagen vitrigel membrane material, and the dried collagen vitrigel membrane material is made easily detachable from the adhesion-preventing sheet when the adhesive film is peeled off while fixing and holding the dried collagen vitrigel membrane material on the surface of the adhesion-preventing sheet during attachment.

Further, the present invention is directed to the patch-type artificial skin preparation wherein the fixing and holding of the dried collagen vitrigel membrane material on the surface of the adhesion-preventing sheet during attachment are achieved by an inner peripheral portion of a window portion of a frame-shaped holding sheet having the window portion, which is provided under the dried collagen vitrigel membrane material and is adhered to the adhesive layer of the adhesive film at an outer peripheral portion thereof, or achieved by protrusions provided therein.

Still further, the present invention is directed to the patch-type artificial skin preparation wherein the fixing and holding of the dried collagen vitrigel membrane material on the adhesion-preventing sheet during attachment are achieved by adhering the dried collagen vitrigel membrane material to the adhesion-preventing sheet with a low adhesive strength.

Yet still further, the present invention is directed to the patch-type artificial skin preparation wherein the fixing and holding of the dried collagen vitrigel membrane material on the adhesion-preventing sheet during attachment are achieved by a physical fixing means between the dried collagen vitrigel membrane material and the adhesion-preventing sheet.

### Advantageous Effects of Invention

The patch-type artificial skin preparation (hereinafter abbreviated as "artificial skin") of the present invention is an adhesive plaster-type preparation integrally including a dried collagen vitrigel membrane material (also referred to as "collagen xerogel membrane") which is a highly cohesive membrane of dense collagen fibers obtained by drying a collagen vitrigel membrane, and has a thickness of about several tens to several hundreds micrometers (µm) and an adhesive film, and therefore, a treatment such as suturing is not required, and the handling thereof is extremely easier than conventional products. Further, when the adhesive film is peeled off after use for a predetermined period of time, the collagen vitrigel membrane is not simultaneously peeled off, so that delay of wound healing by secondary damage due to impairment of cell components which have infiltrated and proliferated inside and outside the vitrigel can be avoided.

As described above, in the artificial skin of the present invention, the dried collagen vitrigel membrane material fixed on this adhesion-preventing film is held during attachment, and also the adhesion-preventing film prevents adhesion between the collagen vitrigel membrane and the adhesive component when the adhesive film is peeled off, and therefore, regenerated tissue including the vitrigel is not damaged. Accordingly, the artificial skin can be easily attached to an area where the skin is lost due to a burn, a decubitus ulcer, traumatic skin loss, or the like, and can be widely used for treating an area where the skin is lost.

### Brief Description of Drawings

[Fig. 1] Fig. 1 is a plan view of an example of a first embodiment of an artificial skin of the present invention viewed from the side to be attached (collagen vitrigel membrane side).
[Fig. 2] Fig. 2 is a view schematically showing a cross section of the artificial skin in Fig. 1. The lower side is the side to be attached.
[Fig. 3] Fig. 3 is a drawing showing a configuration of the artificial skin in Fig. 1 and Fig. 2.
[Fig. 4] Fig. 4 is a drawing showing another example of a frame-shaped holding sheet.
[Fig. 5] Fig. 5 is a drawing showing a configuration of an example of a second embodiment of an artificial skin of the present invention.
[Fig. 6] Fig. 6 is a photograph (x40) of a cross section of regenerated skin tissue after peeling an adhesive film in a present inventive artificial skin attached group.
[Fig. 7] Fig. 7 is a view showing the expression of a myofibroblast marker (α-SMA) in the regenerated skin tissue in Fig. 6. In the vicinity of the collagen vitrigel above the dotted line, myofibroblasts showing high expression of α-SMA are observed, however, in the inside of the collagen vitrigel below the dotted line, many fibroblasts are observed.
[Fig. 8] Fig. 8 is a photograph (x40) of a cross section showing a state of regenerated skin tissue after peeling an adhesive film in a two-layer type group as comparison.
[Fig. 9] Fig. 9 is a drawing showing the expression of TGF-β from fibroblasts. The view on the right side shows a present inventive artificial skin attached group, and the view on the left side shows a two-layer type attached group.
[Fig. 10] Fig. 10 is a drawing showing the expression of CTGF from fibroblasts. The view on the right side shows a present inventive artificial skin attached group, and the view on the left side shows a two-layer type attached group.

### Description of Embodiments

The artificial skin of the present invention is made easily detachable from the adhesion-preventing sheet when the adhesive film is peeled off after the collagen vitrigel membrane is fused to tissue at a wound site while fixing and holding the dried collagen vitrigel membrane material (hereinafter referred to as "dried collagen vitrigel membrane") included in the artificial skin on the adhesion-preventing sheet during attachment.

As one specific example of an embodiment of the artificial skin of the present invention, a patch-type artificial skin preparation which includes an adhesive film, an adhesion-preventing sheet, a dried collagen vitrigel membrane, and a frame-shaped holding sheet in this order, and is characterized in that the adhesion-preventing sheet has substantially the same area as that of the dried collagen vitrigel membrane, and is attached to an adhesive layer of an adhesive tape at a position corresponding to the position of the dried collagen vitrigel membrane, and the frame-shaped holding sheet has a window portion, and the frame-shaped holding sheet is adhered to the adhesive layer of the adhesive film at an outer peripheral portion thereof, and holds the dried collagen vitrigel membrane by an inner peripheral portion of the window portion or protrusions provided therein (hereinafter referred to as "first embodiment invention") can be exemplified.

Further, as an example of another embodiment of the artificial skin of the present invention, a patch-type artificial skin preparation which includes an adhesive film, an adhesion-preventing sheet, and a dried collagen vitrigel membrane in this order, and is characterized in that the adhesion-preventing sheet has substantially the same area as that of the dried collagen vitrigel membrane, and is attached to an adhesive layer of an adhesive tape at a position corresponding to the position of the dried collagen vitrigel membrane, and the dried collagen vitrigel membrane is attached to the adhesion-preventing sheet with a low adhesive strength and held thereon (hereinafter referred to as "second embodiment invention") can be exemplified.

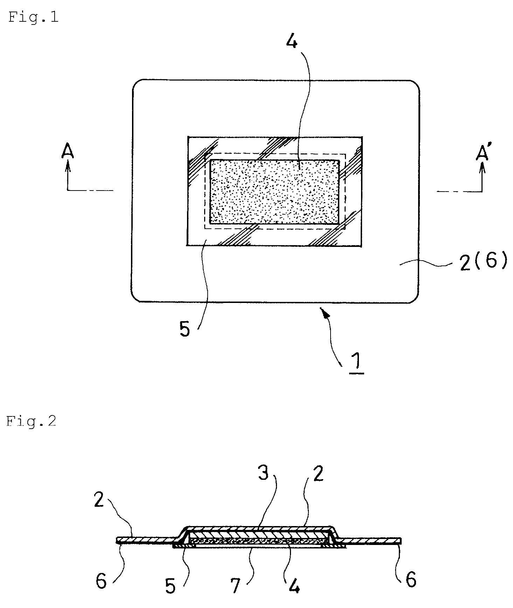

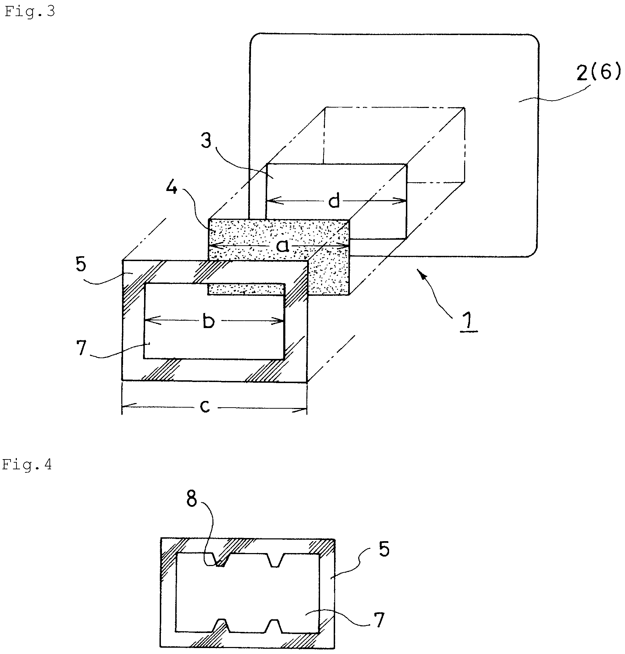

Hereinafter, with respect to the artificial skin of the present invention, the present invention will be described in more detail with reference to the drawings showing several examples of the embodiments. Fig. 1 is a plan view showing the first embodiment invention of the artificial skin of the present invention, Fig. 2 is a cross-sectional view taken along the line A-A' of Fig. 1, and Fig. 3 is a drawing showing the configuration thereof. In the respective drawings, 1 denotes an artificial skin, 2 denotes an adhesive film, 3 denotes an adhesion-preventing sheet, 4 denotes a dried collagen vitrigel membrane, 5 denotes a frame-shaped holding sheet, 6 denotes an adhesive layer, and 7 denotes a window portion.

As shown in Figs. 1 to 3, in the first embodiment invention, the dried collagen vitrigel membrane 4 is disposed on the side of the adhesive layer 6 of the adhesive film 2 interposed by the adhesion-preventing sheet 3.

In the artificial skin 1 of the present invention, the adhesive film 2 which is used as a base body and covers the dried collagen vitrigel membrane 4 is not particularly limited as long as it can be attached to and used for the human skin, and a combination of a base material to be used in a common patch preparation with an adhesive can be used. As one example of such an adhesive film, a transparent dressing tape or the like, in which an adhesive layer 6 is formed by applying an acrylic adhesive or a silicone-based adhesive as an adhesive to one surface of a polyurethane sheet such as a polyether-polyamide copolymer, and the surface thereof on the other side is covered with a protective film of polypropylene or the like, can be exemplified. As such a dressing tape, for example, a commercially available product called "YouTape" (manufactured by Yutoku Pharmaceutical Ind. Co., Ltd.) or the like can also be used.

Further, the dried collagen vitrigel membrane 4 to be used in the artificial skin 1 of the present invention is a dried material of a thin membrane of dense collagen fibers as described above. This material can be prepared by the method described in PTL 1 using atelocollagen derived from an animal such as pigs or cattle as a raw material. As the dried collagen vitrigel membrane 4 to be used in the present invention, a dried atelocollagen vitrigel membrane material containing the above atelocollagen as a raw material is preferred, and particularly, a dried atelocollagen vitrigel membrane material containing type III collagen in an amount of about 1 to 25%, preferably around 10% is preferred because it excels in suppression of scar formation.

In the artificial skin 1 of the present invention, the dried collagen vitrigel membrane 4 can be used by forming it into a desired shape and a desired size. For example, it can be formed into various shapes such as a square, a rectangle, a circle, an ellipse, and a cloud according to the shape of a wound area. Further, its size can also be appropriately determined according to the size of a wound area.

Further, the adhesion-preventing sheet 3 is provided between the dried collagen vitrigel membrane 4 and the adhesive layer 6 of the adhesive sheet 2 so as to prevent the adhesion of the dried collagen vitrigel membrane 4 to the adhesive layer 6 of the adhesive film 2. This sheet itself adheres to the adhesive layer 6 and substantially does not adhere to the dried collagen vitrigel membrane material 4, and can be used without being particularly limited to the material as long as it is a sheet having such a property. As one example of such an adhesion-preventing sheet 3, a plastic sheet subjected to a release treatment on one surface, and as a particularly preferred one, a silicone-coated PET sheet can be exemplified.

This adhesion-preventing sheet 3 has a shape substantially the same as that of the dried collagen vitrigel membrane 4, and may have a size sufficient for preventing the adhesion of the membrane 4 to the adhesive layer of the adhesive film. More specifically, the size (area) thereof is preferably the same as or larger by about 3 to 10% than that of the dried collagen vitrigel membrane material 4.

The reason why the shape and size of the adhesion-preventing sheet 3 are limited in this manner is that if the size thereof too small, in the case where the dried collagen vitrigel membrane 4 protrudes from the adhesion-preventing sheet 3, the dried collagen vitrigel membrane 4 adheres to the adhesive sheet 2 to cause a problem that when the adhesive sheet 2 is peeled off, the membrane 4 is simultaneously peeled off. On the other hand, if the size is too large, in the case where the adhesive film 2 is present in the skin, a contact area with an outer peripheral portion of the below-mentioned frame-shaped sheet 5 is decreased to cause a problem from the viewpoint of adhesiveness.

Further, in the first embodiment invention, the frame-shaped holding sheet 5 is provided on the dried collagen vitrigel membrane 4 on the opposite side from the adhesion-preventing sheet 3. This frame-shaped holding sheet 5 can be formed into the shape of a rectangle, a square, an ellipse, a circle, or the like, and also the window portion 7 can be formed into the shape of a rectangle, a square, an ellipse, a circle, or the like according to the shape of the collagen vitrigel 4.

As one example of the frame-shaped holding sheet 5 with a rectangular shape, as shown in Fig. 1, a sheet which is in the shape having the window portion 7 with a rectangular shape in the center and is adhered to the adhesive layer 6 of the adhesive sheet 2 at an outer peripheral portion thereof is exemplified. This sheet has a function to hold the dried collagen vitrigel membrane 4 by an inner peripheral portion thereof (on the window portion side) as shown in Figs. 1 and 2. That is, fixing in a non-adhesive manner is achieved by fastening and pressing the dried collagen vitrigel membrane 4 with the inner peripheral portion of the frame-shaped holding sheet 5 in a state where the outer peripheral portion of the frame-shaped holding sheet 5 is fixed. Therefore, as described above, the window portion 7 of the frame-shaped holding sheet 5 is required to have a shape similar to the shape of the dried collagen vitrigel membrane material 4.

The holding of the dried collagen vitrigel membrane 4 by the frame-shaped holding sheet 5 is performed in the vicinity (inner peripheral portion) of the window portion 7 smaller than the dried collagen vitrigel membrane 4 in the embodiment in Figs. 1 to 3. Further, in another example of the frame-shaped holding sheet 5, as shown in Fig. 4, a plurality of protrusion portions 8 are provided in the window portion 7 larger than the dried collagen vitrigel membrane 4. The frame-shaped holding sheet 5 of this embodiment can hold the dried collagen vitrigel membrane 4 by the protrusion portions 8.

In the embodiment in Figs. 1 to 3 in which the dried collagen vitrigel membrane 4 is held and fixed by the inner peripheral portion of the window portion 7 described above, the relationship of the sizes of the window portion 7 and the outer frame of the frame-shaped holding sheet 5 and the size of the adhesion-preventing sheet 3 with respect to the size of the dried collagen vitrigel membrane 4 is important.

That is, the length (b) of the corresponding window portion 7 of the frame-shaped holding sheet 5 in Fig. 3 must be shorter than the length (a) of one side of the dried collagen vitrigel membrane 4. This is because it is necessary to prevent the collagen vitrigel membrane 4 from falling off in the inner peripheral portion of the window portion 7 of the frame-shaped holding sheet 5, and the following relationship must surely be satisfied: a>b. Further, as having been described above, the length (c) of a side of an outer periphery of the frame-shaped holding sheet 5 must be longer than the length (a) of the side of the collagen vitrigel membrane 4 because at least a part of the outer periphery is required to be adhered and fixed to the adhesive layer 6 of the adhesive film 2. That is, the following relationship must be satisfied: c>a. Incidentally, in Fig. 3, with respect to the case where the dried collagen vitrigel membrane 4 is in a rectangular shape, only the relationship in a horizontal direction is shown, however, the same relationship is required to be satisfied also in a vertical direction. Further, in the case where the shape is a circle, the same relationship with respect to the diameter thereof is required to be satisfied.

A specific length b with respect to the length a is preferably shorter by about 5 to 15%, although it depends on a material to be used as the frame-shaped holding sheet 5. If the length b is shorter by less than 5%, the dried collagen vitrigel membrane 4 may be liable to fall off when it is attached, and on the other hand, if the length b is shorter by more than 15%, when the adhesive film 2 of the artificial skin is peeled off, this portion is liable to be left in the collagen vitrigel membrane, and therefore, such a length is not preferred. Further, the length c with respect to the length a is desirably larger by about 2 to 5%.

Further, the length (d) of the side of the adhesion-preventing sheet 3 in Fig. 3 is required to be the same as or slightly longer than the length a of the side of the dried collagen vitrigel membrane 4, that is, the following relationship is required to be satisfied: a≤d. It is theoretically possible to prevent adhesion between the adhesive layer of the adhesive film and the dried collagen vitrigel membrane 4 when a=d, however, in the actual production step, some allowance should be made, and therefore, it is preferred to satisfy the following relationship: a<d. The length d in this case suffices as long as it is longer by about 2 to 5% than the length a.

Incidentally, in the case where a sheet in the shape as shown in Fig. 4 is used as the frame-shaped holding sheet 5, it is not necessary to satisfy the above relationship between a and c. However, in this case, at least the distance (e) between the protrusion portions 8 must be smaller than the length a to such an extent that the dried collagen vitrigel membrane 4 can be held.

The frame-shaped holding sheet 5 described above has the above-mentioned holding function, and therefore is desirably a material having a certain degree of strength, and for example, a sheet of PET or the like can be used. Further, it is preferred to perform a release treatment of the sheet 5 on the dried collagen vitrigel membrane 4 side so that the sheet 5 can be easily peeled off from the collagen vitrigel membrane when it is peeled off after use.

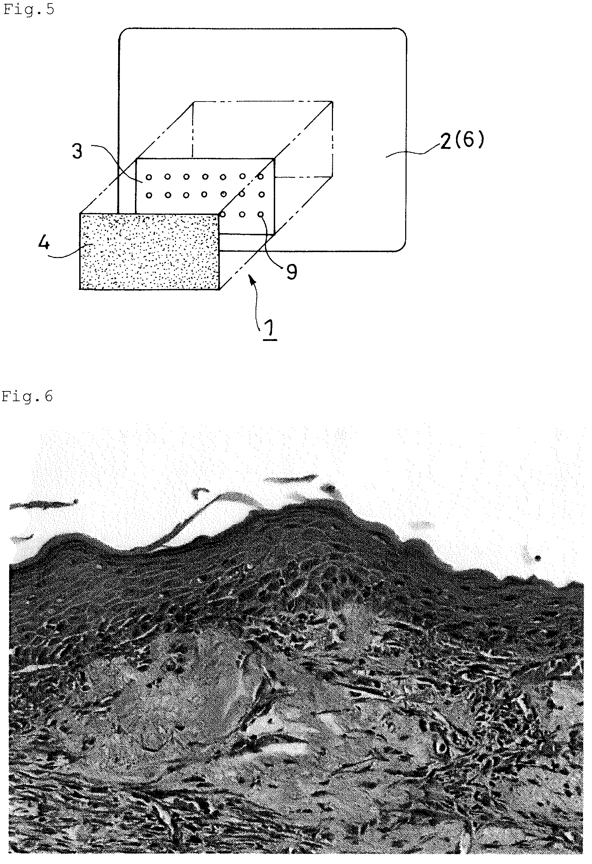

Next, the second embodiment invention of the artificial skin of the present invention will be described with reference to Fig. 5. In Fig. 5, 1 to 4 and 6 denote the same members as in Figs. 1 to 3, and 9 denotes a low-adhesive portion (dot-shaped application portion).

The artificial skin of this embodiment is composed of an adhesive film 2, an adhesion-preventing sheet 3, and a dried collagen vitrigel membrane 4, and does not need a frame-shaped holding sheet 5 which is included in the first embodiment.

In this embodiment, in place of this, a low-adhesive portion 9 having low adhesiveness such that it can hold the dried collagen vitrigel membrane 4 with moderate adhesiveness when it is attached, and also it can be easily detached from the collagen vitrigel membrane when the adhesive film 2 is peeled off is provided on the adhesion-preventing sheet 3 on the side of the dried collagen vitrigel membrane 4.

In Fig. 5, the low-adhesive portion 9 is formed by applying an adhesive in a dot shape, but is not limited thereto, and may be formed by applying an adhesive in a line shape, or may be formed by applying an adhesive to the entire surface when the adhesive has extremely low adhesiveness. In short, the degree of adhesive strength required for the low-adhesive portion 9 may be such that when the artificial skin 1 of the second embodiment is attached to a wound area, the dried collagen vitrigel membrane 4 does not fall off or slip from the adhesion-preventing sheet 3, and when the adhesive film 2 covering the entire membrane is peeled off after the wound is recovered, the collagen vitrigel membrane is extremely easily peeled off from the adhesion-preventing sheet 3. As an example of such an adhesive, preferably, an adhesive having biocompatibility, more preferably a glue-based adhesive (gelatin) with good compatibility with the dried collagen vitrigel material are exemplified. Further, there is also a method in which low adhesiveness is imparted without using an adhesive. That is, it is a method in which a hydrate of collagen vitrigel is stacked on silicone-coated PET, followed by drying. Both can maintain moderate adhesiveness.

Further, as a modification of the second embodiment, a patch-type artificial skin preparation in which the fixing and holding of the dried collagen vitrigel membrane 4 on the adhesion-preventing sheet 3 are achieved by a physical fixing means between the dried collagen vitrigel membrane 4 and the adhesion-preventing sheet 3 can be exemplified (not shown).

In this embodiment, the dried collagen vitrigel membrane 4 and the adhesion-preventing sheet 3 are press-bonded, fixed, and held by a physical fixing means, for example, needle puncture with a plurality of needles or the like, partial compression with a mold having a plurality of protrusions, or the like in place of the low-adhesive portion 9 having low adhesiveness such that it can hold the dried collagen vitrigel membrane 4 when it is attached, and also it is easily detached from the collagen vitrigel membrane when it is peeled off in the second embodiment. Also in this case, in the same manner as in the second embodiment, the degree of fixing strength may be such that when the artificial skin 1 is attached to a wound area, the collagen vitrigel membrane 4 does not fall off or the like from the adhesion-preventing sheet 3, and when the adhesive film 2 is peeled off, the collagen vitrigel membrane is extremely easily peeled off from the adhesion-preventing sheet 3.

The size of the artificial skin 1 of the present invention described above is not particularly limited, however, for example, in the case where the shape thereof is assumed to be a commonly used rectangle, it is preferred that the entire size (the size of the adhesive film 2) is about 60 to 200 mm x 60 to 220 mm, the size of the dried collagen vitrigel membrane 4 to be used therefor is 25 to 50 mm x 25 to 50 mm, the size of the outer shape of the frame-shaped holding sheet 5 is about 30 to 60 mm x 30 to 60 mm. Further, the same shall apply also to the case of another shape such as a circle.

The artificial skin 1 of the present invention can be processed into a final form in the end by bonding a release paper (not shown) or the like subjected to a release treatment by silicone coating or the like on the inside thereof to the entire surface of the adhesive layer 6 of the adhesive film 2 so as to cover the dried collagen vitrigel membrane 4, and if present, the frame-shaped holding sheet 5. By bonding a release paper in this manner, the adhesive layer 6 of the adhesive film 2 is protected, and also in the case where the frame-shaped holding sheet 5 is present, the sheet 5 is made difficult to move, and thus, the dried collagen vitrigel membrane 4 can be stably held.

Then, the artificial skin 1 in the final form can be processed into a final product by, for example, packaging it with sterile paper, followed by sterilization, and then, enclosing it in an aluminum pack or the like. Incidentally, as the sterilization method, EOG (ethylene oxide gas) sterilization or electron beam sterilization can be performed. In comparison between EOG sterilization and electron beam sterilization, it has been confirmed that the wound area reduction ratio is equivalent, however, the tissue remaining ratio is higher in the case of EOG sterilization, and therefore EOG sterilization is superior. Further, the myofibroblast appearance ratio and the cell migration ratio are higher in the case of EOG sterilization than in the case of electron beam sterilization, and it has been confirmed that EOG sterilization is superior in that the appearance of myofibroblasts is suppressed. Therefore, the sterilization method is preferably EOG sterilization.

The artificial skin of the present invention described above can be easily attached to a wound area where the skin is lost due to a burn, a decubitus ulcer, traumatic skin loss, or the like, and suppresses scar formation and promotes skin regeneration by suppressing the appearance of myofibroblasts while maintaining a moist environment at the wound site by the adhesive film attached and supplying a scaffold to epidermal cells from a collagen component contained in the dried collagen vitrigel membrane 4.

Then, also when the adhesive film 2 is peeled off from the wound area, the adhesion-preventing sheet 3 is present between the collagen vitrigel membrane and the adhesive layer 6 of the adhesive film 2, and therefore, the collagen vitrigel membrane remains in the wound area without adhering to the adhesive film 2, whereby secondary damage can be prevented.

The artificial skin of the present invention having such a function provides a new treatment method for wounds and can be expected to be widely used. One example of a specific usage of the artificial skin of the first embodiment invention is as follows.

### [Preparation before Use]

(1) Open the outer package in a clean environment and take out the sterile paper package from the aluminum pack.
(2) Aseptically take out the main body from the sterile paper and place the main body in a sterile container.
(3) Prepare an appropriate number of preparations corresponding to the size of vitrigel which fits to the size of an affected area to be treated.

### [Attachment Method]

(1) After sufficiently perform debridement of the affected area, peel off the release paper on the dried collagen vitrigel membrane side, and the surface thereof is applied to the affected area and attach it so as to cover the affected area.
(2) Aseptically peel off the polypropylene film present on the upper part of the adhesive film while pressing the film so as not to move in a state where the entire affected area is covered, and firmly press the film.
(3) Confirm that the entire affected area is in a state of being covered with the dried collagen vitrigel membrane, and also confirm that a moist environment is formed.
(4) Confirm that an air bubble is not present between the artificial skin of the present invention and the wound surface. If an air bubble is present, guide the air bubble to the outside with a finger.
(5) In the case where wetting fluid overflows and the adhesive film does not adhere, perform hemostasis and debridement again, and apply the dried collagen vitrigel membrane again.
(6) Find the timing of peeling while observing the conditions of the affected area for 1 to 2 weeks. Remove all of the frame-shaped holding sheet which plays a role in pressing, the adhesion-preventing sheet, and the adhesive film and leave only the collagen vitrigel thin membrane on the affected area.

### Examples

Next, the present invention will be described in more detail by showing Examples, however, the present invention is by no means limited to these Examples.

### Example 1

### Effect of Treating Massive Skin Defects

All layers of the skin in a wide range with a diameter of 1.5 cm in a dorsal area of each wild-type mouse (C57BL6J) were excised, whereby a skin loss wound model was created. To the wound area, the artificial skin of the present invention or a covering material was attached, and the inhibitory effect on scar contraction in the wound area was examined.

The artificial skin of the present invention is a material of a three-layer type having a configuration as shown in Figs. 1 and 2, and the dried collagen vitrigel membrane used was obtained from National Institute of Agrobiological Sciences, and in the shape of a circle with a diameter of 1.5 cm and having a thickness of about 70 to 100 µm. Further, as the frame-shaped holding sheet, a PET sheet with a diameter of 1. 8 cm in which a window with a diameter of 1. 3 cm was opened was used. As the adhesion-preventing sheet, a silicone-treated PET sheet with a diameter of 1. 6 cm was used. Further, as the adhesive film, a dressing film (YouTape (manufactured by Yutoku Pharmaceutical Ind. Co., Ltd.), 6 x 10 cm) was used.

On the other hand, as the covering material for comparison, a material of a two-layer type including the above-mentioned dressing film and adhesion-preventing sheet in combination (a two-layer type attached group) was used, and further, none was attached for comparison (a non-attached group).

The size of the wound area in the skin wound defect model was observed over time from the start of the test, and the wound area reduction ratio was examined. As a result, in comparison with the non-attached group and the two-layer type attached group, in the case where the artificial skin of the present invention of a three-layer type was attached (the present inventive artificial skin attached group), a significant difference was not observed until a reduction ratio reaches to 90% at which complete healing was determined.

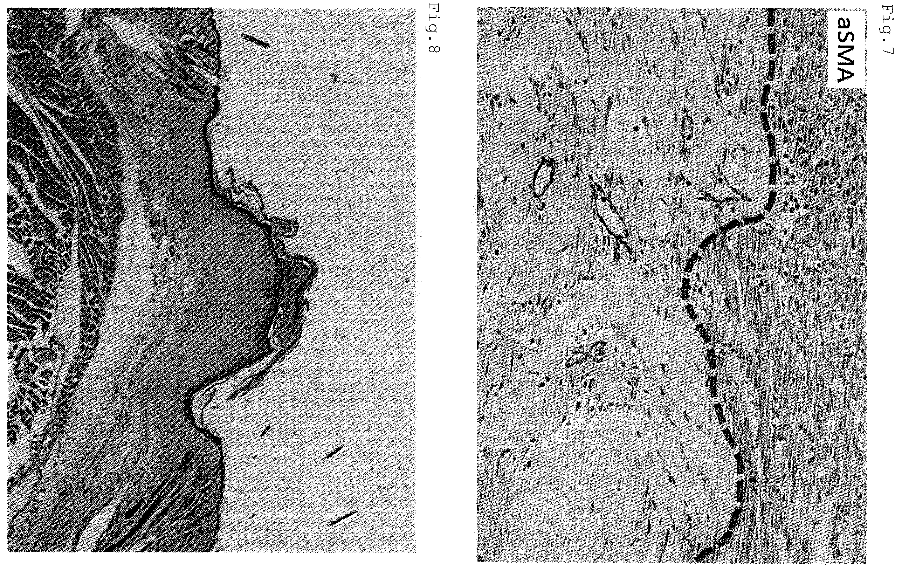

Further, in the present inventive artificial skin attached group, the regenerated skin tissue after the adhesive film was peeled off had a flat shape macroscopically (Fig. 6). Further, when a histopathological analysis was performed, a large amount of residual vitrigel was found in the wound area, and infiltration of spindle cells into the vitrigel was observed. However, a xenobiotic reaction with the vitrigel left in the tissue or infiltration of CD68-positive macrophages was not observed. Further, spindle cells proliferating in the vicinity of the vitrigel showed high expression of a myofibroblast marker (α-SMA), however, myofibroblasts inside the vitrigel were very few, and its appearance ratio corresponded approximately to fibroblasts (Fig. 7).

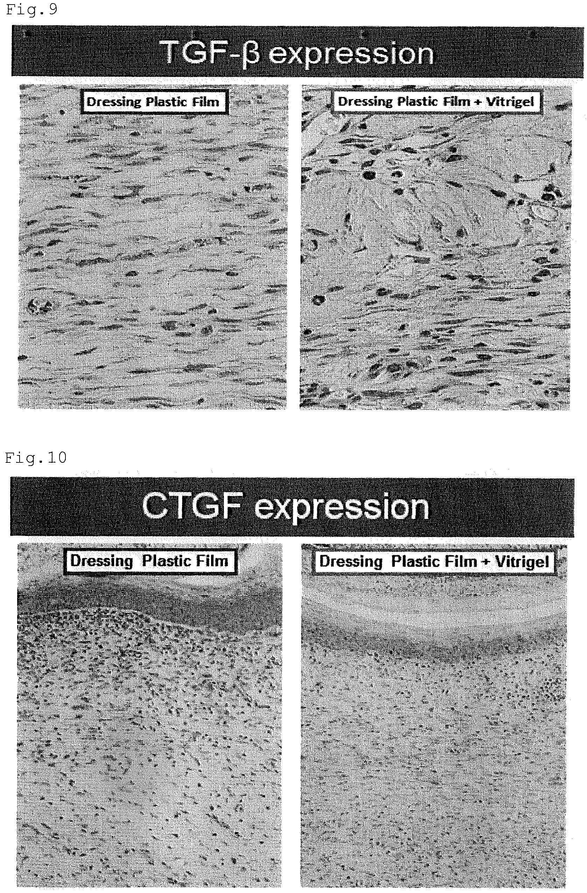

On the other hand, in the two-layer type attached group, the formation of a pathological protruded scar was observed in the regenerated skin (Fig. 8), and almost all the spindle cells appearing in the regenerated skin tissue were α-SMA-positive myofibroblasts. Further, in comparison with the present inventive artificial skin attached group, in the two-layer type attached group, the expression of TGF-β (left side of Fig. 9) and CTGF (left side of Fig. 10) from fibroblasts was higher, and thus, the phenomenon of strong pathological fibril formation in the wound area could be confirmed also molecular cytologically.

Based on the above results, it was considered that vitrigel suppresses the transformation of fibroblasts to myofibroblasts, and it was presumed that this inhibitory effect on the transformation contributes to the fibril formation for wound closure and the inhibitory effect on scar formation.

### Industrial Applicability

We developed an artificial skin having performance equal to or greater than the conventional products by inserting an adhesion-preventing sheet such as a silicone-treated PET film between an adhesive film such as a dressing tape and a dried vitrigel membrane.

A collagen portion of a currently commercially available artificial skin is processed into a sponge form, and suturing is required for fixing it to a wound area, and therefore, it has a drawback in terms of handleability. On the other hand, the dried collagen vitrigel membrane to be used in the present invention is a dense collagen formed into a thin membrane by a vitrification treatment, and is totally different and new as a material. Then, by combining this with an adhesive film such as a dressing tape, suturing is no longer needed, and thus the handleability is dramatically improved.

Then, the adhesive film to be used in the artificial skin of the present invention maintains a moist environment at a wound site and forms a physical barrier from the external environment. Further, the adhesion-preventing sheet prevents the adhesion of the collagen vitrigel membrane and regenerated tissue to the adhesive film during dressing change or the like, and prevents secondary damage.

Further, from the medical viewpoint, the collagen vitrigel membrane supplies collagen to a wound area and regulates the microenvironment in tissue regeneration, and in addition thereto, promotes regeneration and at the same time suppresses pathological scar formation by the inhibitory effect on the appearance of myofibroblasts of its own. Moreover, the dried collagen vitrigel membrane to be used in the present invention has very high biocompatibility, and it has not been confirmed that it causes a rejection response or a xenobiotic reaction in a wound area.

Accordingly, the artificial skin of the present invention can be used by a simple means such as attachment thereof to an area where the skin is lost due to a burn, a decubitus ulcer, traumatic skin loss, or the like, and also can be expected to have an effect of occlusive dressing, and therefore can be widely used for treating an area where the skin is lost caused by various factors. In particular, in a currently available artificial skin product, replacement (dressing change) accompanying contamination cannot be performed basically, however, in the product of the present invention, since the adhesion-preventing sheet is adopted, dressing change which is general in the treatment of wound area can also be performed and thus, the product of the present invention is very advantageous also from the viewpoint of infection control measures.

### Reference Sings List

- 1:: artificial skin
- 2:: adhesive film
- 3:: adhesion-preventing sheet
- 4:: dried collagen vitrigel membrane
- 5:: frame-shaped holding sheet
- 6:: adhesive layer
- 7:: window portion
- 8:: protrusion portion
- 9:: low-adhesive portion

## Claims

1. A patch-type artificial skin preparation comprising at least an adhesive film, an adhesion-preventing sheet, and a dried collagen vitrigel membrane material in this order, **characterized in that** the adhesion-preventing sheet has a sufficient size for preventing the adhesion of the dried collagen vitrigel membrane material to an adhesive layer of the adhesive film and is attached to the adhesive layer of the adhesive film at a position corresponding to the position of the dried collagen vitrigel membrane material, and the dried collagen vitrigel membrane material is made easily detachable from the adhesion-preventing sheet when the adhesive film is peeled off while fixing and holding the dried collagen vitrigel membrane material on the surface of the adhesion-preventing sheet during attachment.

2. The patch-type artificial skin preparation according to claim 1, **characterized in that** the fixing and holding of the dried collagen vitrigel membrane material on the surface of the adhesion-preventing sheet during attachment are achieved by an inner peripheral portion of a window portion of a frame-shaped holding sheet having the window portion, which is provided under the dried collagen vitrigel membrane material and is adhered to the adhesive layer of the adhesive film at an outer peripheral portion thereof, or achieved by protrusions provided therein.

3. The patch-type artificial skin preparation according to claim 1, **characterized in that** the fixing and holding of the dried collagen vitrigel membrane material on the surface of the adhesion-preventing sheet during attachment are achieved by attaching the dried collagen vitrigel membrane material to the adhesion-preventing sheet with a low adhesive strength.

4. The patch-type artificial skin preparation according to any one of claims 1 to 3, wherein the adhesion-preventing sheet is a plastic sheet subjected to a release treatment on one surface.

5. The patch-type artificial skin preparation according to claim 4, wherein the plastic sheet subjected to a release treatment is a silicone-coated polyethylene terephthalate sheet.

6. The patch-type artificial skin preparation according to any one of claims 1 to 5, wherein the dried collagen vitrigel membrane material is a dried atelocollagen vitrigel membrane material.

7. The patch-type artificial skin preparation according to any one of claims 1 to 6, **characterized in that** the preparation can be easily attached to an area where the skin is lost, and even when the preparation is replaced, the collagen vitrigel membrane remains at a wound site without adhering to the adhesive film and the adhesion-preventing sheet so as to impart a function to prevent secondary damage.

8. The patch-type artificial skin preparation according to any one of claims 1 to 7, **characterized in that** scar formation is suppressed and fast healing is achieved by suppressing the appearance of myofibroblasts while maintaining a moist environment at the wound site by the adhesive film and supplying a scaffold to epidermal cells from a collagen component contained in the dried collagen vitrigel membrane material.

9. The patch-type artificial skin preparation according to any one of claims 1 to 8, **characterized in that** the dried collagen vitrigel membrane material is formed from atelocollagen containing type III collagen, and the collagen vitrigel membrane remains for a long period of time also after it is transferred to the wound area, and does not cause a xenobiotic reaction.

10. The patch-type artificial skin preparation according to claim 9, **characterized in that** the transdifferentiation of fibroblasts to myofibroblasts is suppressed by suppressing the expression of TGF-β and CTGF in fibroblasts in the vicinity of the wound site by the effect of type III collagen contained in the collagen vitrigel.
